# EUROPÄISCHE PATENTSCHRIFT

(11) **EP 1 799 189 B1**
(45) Veröffentlichungstag und Bekanntmachung des Hinweises auf die Patenterteilung: **12.05.2010**
(21) Anmeldenummer: 05795638.5
(22) Anmeldetag: 07.10.2005
(51) Int. Cl.: A61K 8/73, A61Q 15/00

(54) **KOSMETISCHE FORMULIERUNG ENTHALTEND CHITOSAN**
CHITOSAN-CONTAINING COSMETIC FORMULATION
FORMULATIONS COSMETIQUES CONTENANT DU CHITOSANE

(30) Priorität: 09.10.2004 DE 102004049282
(43) Veröffentlichungstag der Anmeldung: 27.06.2007
(73) Patentinhaber: Beiersdorf Aktiengesellschaft, 20253 Hamburg (DE)
(72) Erfinder: MEIER-ZIMMERER, Cornelia, 22529 Hamburg (DE); KUX, Ulrich, 22559 Hamburg (DE); CIERPISZ, Yvonne, 20251 Hamburg (DE); FOELSTER, Heike, 20257 Hamburg (DE); MEYER, Maren, 22459 Hamburg (DE); KRISTOF, Boris, 25469 Halstenbek (DE); RUPPERT, Stephan, 20259 Hamburg (DE); URBAN, Michael, 22393 Hamburg (DE); ROHDE, Claudia, D-25499 Tangstedt (DE); ENGFELDT, Linda, 22047 Hamburg (DE); HALLMANN, Marita, 22417 Hamburg (DE); MAURER, Peter, 24536 Neumünster (DE)
(86) Internationale Anmeldenummer: PCT/EP2005/010831
(87) Internationale Veröffentlichungsnummer: WO 2006/040091

(56) Entgegenhaltungen:
- EP-A- 0 377 091
- EP-A- 0 664 301
- WO-A-97/16164
- WO-A-98/15255
- DE-A1- 10 014 529
- DE-A1- 10 145 111
- US-A- 5 015 632
- US-A- 5 077 052
- US-A1- 2003 133 891

## Beschreibung

Die Erfindung betrifft eine kosmetische Formulierung mit einem Anteil an Chitosan.

Vor allem aus ästhetischen Gründen werden transparente und transluzente kosmetische Produkte von vielen Verbrauchern bevorzugt. Transparente Formulierungen kommen so z. B. häufig als Deo oder Antitranspirant (AT) zum Einsatz. Diese lassen sich heutzutage durch folgende Technologien realisieren:
1. wässrig-alkoholische Formulierungen
2. Wasser-in-Silikon-Emulsionen
3. Mikro-Emulsionen
4. Nano-Emulsionen

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien (AT) kann durch Adstringentien - vorwiegend Aluminiumsalzen wie Aluminiumhydroxychlorid (Aluminiumchlorhydrat, ACH oder aktiviertes ACH - AACH) oder Aluminium-Zirkonium-Salze (AZG) die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflusst, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

Übliche, und sich gerade in neuerer Zeit immer weiter verbreitende kosmetische Zubereitungsformen sind Gele.

Im technischen Sinne werden unter Gelen verstanden: relativ formbeständige, leicht verformbare disperse Systeme aus zumindest zwei Komponenten, welche in der Regel aus einem - meist festen - kolloid zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h. ein vorwiegend polares (insbesondere: hydrophiles) Geliermittel geliert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrükkenbindungen zwischen Geliermittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca. 0,2 - 1,0 % eines Geliermittels) und dabei durchaus feste Konsistenz besitzen. Der Wasseranteil liegt dabei in eisähnlichen Strukturelementen vor, so dass Gele daher ihrer Namensherkunft [aus lat. "gelatum" = "Gefrorenes" über den alchimistischen Ausdruck "gelatina" (16. Jhdt.) für nhdt. "Gelatine"] durchaus gerecht werden.

In der kosmetischen und pharmazeutischen Galenik sind ferner auch Lipogele und Oleogele (aus Wachsen, Fetten und fetten Ölen) sowie Carbogele (aus Paraffin oder Petrolatum) geläufig. In der Praxis unterscheidet man Oleogele, welche praktisch wasserfrei vorliegen, Hydrogele, welche praktisch fettfrei sind und Öl/Wasser-Gele, welche letztlich auf O/W- oder W/O-Emulsionen basieren, in welchen zusätzlich aber auch Merkmale einer Gelstruktur verwirklicht sind. Meistens sind Gele durchsichtig. In der kosmetischen bzw. pharmazeutischen Galenik zeichnen sich Gele in aller Regel durch halbfeste, oft fließfähige Konsistenz aus.

In einfachen Emulsionen liegen in der einen Phase feindisperse, von einer Emulgatorhülle umschlossene Tröpfchen der zweiten Phase (Wassertröpfchen in W/O-oder Lipidvesikel in O/W-Emulsionen) vor. Die Tröpfchendurchmesser der gewöhnlichen Emulsionen liegen im Bereich von ca. 1 µm bis ca. 50 µm. Solche "Makroemulsionen" sind, ohne weitere färbende Zusätze, milchig weiß gefärbt und opak. Feinere "Makroemulsionen", deren Tröpfchendurchmesser im Bereich von ca. 10⁻¹ µm bis ca. 1 µm liegen, sind, wiederum ohne färbende Zusätze, bläulich weiß gefärbt und undurchsichtig.

Mizellaren und molekularen Lösungen mit Partikeldurchmessern kleiner als ca. 10⁻² µm, ist vorbehalten, klar und transparent zu erscheinen.

Der Tröpfchendurchmesser von transparenten bzw. transluzenten Mikro- und Nanoemulsionen dagegen liegt im Bereich von etwa 10⁻² µm bis etwa 10⁻¹ µm. Solche Mikroemulsionen sind meist niedrigviskos. Die Viskosität vieler Mikroemulsionen vom O/W-Typ ist vergleichbar mit der des Wassers.

Vorteil von Mikroemulsionsgelen ist, dass in der dispersen Phase Wirkstoffe feindispers vorliegen können. Ein weiterer Vorteil ist, dass sie aufgrund ihrer niedrigen Viskosität versprühbar sein können. Werden Mikroemulsionen als Kosmetika verwendet, zeichnen sich entsprechende Produkte durch hohe kosmetische Eleganz aus.

Es ist an sich bekannt, die Tröpfchen einer niedrigviskosen, insbesondere dünnflüssigen Mikroemulsion mit vernetzenden Substanzen miteinander zu verknüpfen, um auf diese Weise das dreidimensionale Netzwerk eines Geles zu erhalten.

In Nachr. Chem. Techn. Lab. 43 (1995) Nr. 1, S. 9 ff. werden zur Vernetzung von Mikroemulsionströpfchen kettenförmige, hydrophile Moleküle beschrieben, welche an den beiden Kettenenden je einen hydrophoben Rest aufweisen. Jene hydrophoben Reste tauchen in Mikroemulsionströpfchen ein, wobei die hydrophilen Kettenbereiche in der kontinuierlichen Wasserphase vorliegen. Im strengen Sinne ist es wohl nicht nötig, dass die hydrophoben Reste "eintauchen". Es kann im Einzelfalle auch durchaus genügen, wenn durch hydrophobe Wechselwirkung die hydrophoben Reste mit der Oberfläche der Mikroemulsionströpfchen in Kontakt treten und mehr oder weniger stark an dieser haften bleiben.

Als Vernetzer werden a.a.O. Polyoxyethylenglycole mit Oleylgruppen als hydrophobe Endgruppen angegeben.

Mikroemulsionsgele eignen sich auch für kosmetische Anwendungen, beispielsweise Desodorantien, so dass die vorliegende Erfindung in einer besonderen Ausführungsform kosmetische Desodorantien betrifft.

Auch die Verwendung von Mikro- und Nanoemulsionen als Grundlage für desodorierende oder antitranspirant wirkende Zubereitungen sind bekannt.

Mikroemulsionen haben den großen Vorteil, dass man auch größere Mengen an verschiedenen Ölen - mit all den beschriebenen positiven Effekten für den Verbraucher - stabil einarbeiten kann. Formulierungen dieser Art sind prinzipiell mittels Phaseninversionstemperatur-Technologie (PIT) oder Hochdruckhomogenisierung zugänglich. Die notwendige Stabilität des Emulgatorsystems gegenüber hohen Konzentrationen an Antitranspirantsalzen stellt jedoch hohe Anforderungen an die Formulierungskunst des Produktentwicklers. WO 98/15255 beschreibt beispielsweise solche Mikro- und Nanoemulsionen.

Eine Aufgabe der vorliegenden Erfindung war es, Zubereitungen zu entwickeln, welche als Grundlage Mikro-, Nano- und/oder Makroemulsionen darstellen und die die Nachteile des Standes der Technik nicht aufweisen.

Weiterhin war es eine Aufgabe der Erfindung, kosmetische Grundlagen für kosmetische Antitranspirantien bzw. Desodorantien zu entwickeln, die sich durch gute Hautverträglichkeit auszeichnen.

In EP 1190702 A1, DE 10014529, EP 857057 und DE 4442987 werden Gele, Emulsionen und desodorierende Zubereitungen offenbart, die Chitosane enthalten.

Chitosane stellen partiell deacetylierte Chitine unterschiedlichen Molekulargewichtes dar, die den idealisierten-Monomerbaustein enthalten. Im Gegensatz zu den meisten Hydrokolloiden, die im Bereich biologischer pH-Werte negativ geladen sind, stellen die einzusetzenden Chitosane unter diesen Bedingungen kationische Verbindungen dar. Die positiv geladenen Chitosane können mit entgegengesetzt geladenen Oberflächen in Wechselwirkung treten und werden daher üblicherweise in kosmetischen Haar- und Körperpflegemitteln eingesetzt. Übersichten zu diesem Thema sind beispielsweise von B.Gesslein et al. in HAPPI 27, 57(1990), O.Skaugrud in Drug Cosm. Ind. 148, 24(1991) und E.Onsoyen et al.in Seifen-Öle-Fette-Wachse 117, 633 (1991) erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei üblicherweise zunächst durch Zusatz von Basen deproteiniert, durch Zugabe von Mineralsäuren demineralisiert und schließlich durch Zugabe von starken Basen deacetyliert.

Neben den bekannten Herstellverfahren der Chitosane maritimen Ursprungs durch nass-chemische Synthesen sind neuerdings auch Verfahren bekannt, in denen durch gentechnisch modifizierte Mikroorganismen enzymatisch hergestelltes Chitosan gewonnen wird. Die Herstellverfahren laufen entsprechend der bekannten Biotechnologie ggf. über Fermentationsprozesse ab.

EP 857057 offenbart desodorierende Zubereitungen, enthaltend Chitosan, Aluminiumchlorhydrat und Esteraseinhibitoren zu denen insbesondere auch Dicarbonsäuren und deren Ester, wie beispielsweise Glutarsäure, Glutarsäuremonoethylester, Glutarsäurediethyl-ester, Adipinsäure, Adipinsäuremonoethylester, Adipinsäurediethylester, Malonsäure und Malonsäurediethylester, Hydroxycarbonsäuren und deren Ester wie beispielsweise Citronensäure, Apfelsäure, Weinsäure oder Weinsäurediethylester zählen.

DE 10014529 offenbart desodorierende Zubereitungen mit nanoskaligen Chitosanen, mit einem Durchmesser von 1 bis 300 nm. Die Chitosane besitzen hierin ein durchschnittliches Molekulargewicht von 50.000 bis 2.000.000 Dalton, eine Viskosität nach Brookfield von weniger als 5.000 mPas und einen Deacetylierungsgrad im Bereich von 80 bis 88%.

EP 1190702 offenbart Chitosan haltige Emulsionen, wobei die hierin eingesetzten Chitosane einen Deacetylierungsgrad von mindestens 70%, bevorzugt wenigstens 90%, eine Viskosität von weniger als 30 mPas und ein geringes Molekulargewicht aufweisen.

DE 4442987 offenbart kosmetische Mittel enthaltend kationische Biopolymere, Chitosane, mit einem durchschnittlichen Molekulargewicht von 10.000 bis 2.000.000 Dalton, einer Viskosität nach Brookfield (1 Gew.-%ig in Glycolsäure) im Bereich von 20.000 bis 30.000 mPas.

Gegenstand der Schrift "Chitin, Chitosan, and Related Enzymes" (Ed. John P.Zikakis, New York, Academic Press, 1984, S. XVII bis XXIV sowie S. 239 bis 255) sind Abbauprodukte des Chitins beschrieben, die einen Deacetylierungsgrad besitzen, der mit nur 17,1 % äußerst niedrig liegt. Derartige Produkte sind in organischen Säuren jedoch völlig unlöslich. Aus FR-A 2701266 sind ebenfalls Abbauprodukte bekannt, die man erhält, indem man Chitin zunächst mit Salzsäure vorbehandelt und anschließend mit Natronlauge so weit als möglich deacetyliert. Es werden Produkte mit einem Deacetylierungsgrad von 92% erhalten, die sich durch einen sehr niedrigen Calciumcarbonatgehalt auszeichnen, in organischen Säuren gut löslich sind und dabei niedrigviskose Produkte ergeben. Von entscheidendem Nachteil ist jedoch wieder, das infolge der drastischen Abbaubedingungen das Molekulargewicht sehr niedrig ist und auch hier wieder die filmbildenden Eigenschaften der Produkte unbefriedigend sind.

Zusammenfassend kann festgestellt werden, das man die kationischen Biopolymere des Stands der Technik in zwei Gruppen einteilen kann: Zur ersten Gruppe von Produkten gehören diejenigen, die einen hohen Deacetylierungsgrad besitzen, in organischen Sauren löslich sind und dabei niedrigviskose Lösungen bilden, jedoch über keine ausreichenden filmbildenden Eigenschaften verfügen. In die zweite Gruppe gehören Produkte, die nur zu einem geringen Anteil deacetyliert worden sind, ein höheres Molekulargewicht und gute filmbildende Eigenschaften aufweisen, jedoch in organischen Säuren allenfalls schwerlöslich und daher nicht konfektionierbar sind. Weiterhin weisen die Produkte des Stands der Technik eine Reihe von weiteren Nachteilen auf: Sie sind in der Regel als Folge des drastischen Abbaus stark verfärbt, geruchlich nicht akzeptabel und wenig lagerstabil, d.h. die Viskositat bleibt bei längerer Lagerzeit nicht erhalten, sondern nimmt ab. Weiterhin ist der Zusatz von Konservierungsmitteln erforderlich, wenn auch nicht gerne gesehen, da die Produkte anfällig gegenüber einer Kontamination durch Mikroorganismen sind.

Des weiteren weisen Chitosanhaltige kosmetische Zubereitungen des Standes der Technik verschiedenste Nachteile auf. Der Einsatz von Chitosanen mit einem hohen Deacetylierungsgrad von mehr als 80% in Nano-, Mikro- oder Makroemulsionen führt zu Instabilitäten, Trübungen, Phasentrennung und/oder Wasserabscheidungen. Dies wird vermutlich durch emulgierende Eigenschaften des Chitosans verursacht; somit kann es zu unerwünschten Wechselwirkungen zwischen Chitosan und den Emulgatoren und emulgierten Ölen bzw. Lipiden in Emulsionen kommen. Durch die naturgemäß gegebene Molekular- und Kettenlängenverteilung des Chitosan kann man Chitosan auch als Mischemulgator ansehen, der aber keinen definierten HLB-Wert, sondern eher einen HLB-Wertebereich besitzt. Die Unterdrückung bzw. Minimierung der emulgierenden

EP 377091 A1 offenbart kosmetische Präparate umfassend Chitosan oder Chitosanderivate mit
- einem Deacetylierungsgrad von 70% bis 95%.
- einer Viskosität von 1 bis 80 mPas (1%ige Lösung)
- einem Gewichtsmittel der Molekulargewichtsverteilung von 3.000 bis 700 000 Da.
   US 5077052 offenbart Präparate umfassend Chitosan oder Chitosanderivate mit
- einem Deacetylierungsgrad über 80% und
- einem Gewichtsmittel der Molekulargewichtsverteilung von 10.000 bis 110.000 Da.
   US 2003/0133891 A1 offenbart kosmetische Präparate umfassend Chitosan oder Chitosanderivate mit
- einem Deacetylierungsgrad von 80% bis 88%,
- einer Viskosität von kleiner 5000 mPas (1%ige Lösung)
- einem Gewichtsmittel der Molekulargewichtsverteilung von 50.000 bis 2.000.000 Da.

Die Zubereitungen können neben anderen üblichen kosmetischen Zusatzstoffen auch ein oder mehrere Antitranspirant-Wirkstoffe aus der Gruppe der Aluminium-Salze enthalten.

Eigenschaften des Chitosans ist eines der zu lösenden Hauptprobleme bei dessen Einarbeitung in Emulsionen.Aufgabe der vorliegenden Erfindung ist es daher auch stabile Deo/AT-Formulierungen mit Chitosan bereit zu stellen, in denen das kationische Biopolymer Chitosan in Mikro-, Nano- als auch in Makroemulsionen stabil eingearbeitet werden kann. Insbesondere ist es die Aufgabe der vorliegenden Erfindung chitosanhaltige und transparente, transluzente sowie opaque desodorierende Zubereitungen bereit zu stellen, d.h. ganz speziell auch Deo- oder Antitranspirantformulierungen bereit zu stellen, die transparent sind und keinerlei Eintrübung aufweisen.

Darüber hinaus ist es die Aufgabe der vorliegenden Erfindung alternative kosmetische Zubereitungen neben den bekannten Zubereitungen mit Chitosanen zur Verfügung zu stellen.

Nach dem Stand der Technik werden Chitosane in verschiedenste kosmetische Formulierungen eingesetzt. Die beispielsweise in DE 10208550 oder EP 1384404 beschriebenen Formulierungen umfassen Chitosane mit einem Molekulargewicht von 800.000 bis 1.200.000 Da bzw. Oligosaccharide mit 1 bis 50 Monomeren und einem Molekulargewicht unter 10.000 Da. Chitosane in diesen Bereichen weisen keine anti-adhäsive Wirksamkeit, insbesondere gegen gram positive Bakterien, auf und zeigen insbesondere in kosmetischen Formulierungen keine schweißgeruchsreduzierende Wirkung.

Aufgabe der vorliegenden Erfindung ist es daher auch eine Formulierung zur Verfügung zu stellen, die gegen die kosmetisch unerwünschten Phänomene, die durch Mikroorganismen verursacht werden, wie Schweißgeruch, Körpergeruch, Fußgeruch, atopisches Ekzem etc. wirksam ist, dabei eine besonders milde aber effiziente Wirksamkeit als bisherige wirkstoffhaltige Zubereitungen aufweist und stabil in Emulsionen eingearbeitet werden kann.

Gelöst wird das Bündel an Aufgaben durch eine kosmetische Formulierung entsprechend Anspruch 1. Gegenstand der Unteransprüche sind vorteilhafte Ausführungsformen der erfindungsgemäßen Zubereitung. Des weiteren umfasst die Erfindung die Verwendung derartiger Zubereitungen sowie die Verwendung der Kombination aus Chitosan und Aluminium-Chlorohydrat zur Herstellung einer stabilen, opaken oder transparenten oder transluzenten desodorierenden Zubereitung.

Es war überraschend und für den Fachmann nicht vorauszusehen, dass eine kosmetische Formulierung umfassend Chitosan oder Chitosanderivate mit
- A: einem Deacetylierungsgrod von 75% bis 98%, bevorzugt 78 bis 86%, insbesondere 75 bis 85%. 82 bis 85% oder 82 bis 84%, sowie 80 bis 98%, bevorzugt 92 bis 98%.
- B: einer Viskosität bis zu 10 mPas, insbesondere von 4 bis 10 mPas, bevorzugt 5 bis 8 mPas,
- C: einem Gewichtsmittel der Molekulargewichtsverteilung von 10.000 bis 300.000 Da, bevorzugt weniger als 160.000 Dalton oder weniger als 150.000 Da, insbesondere bevorzugt weniger als 100.000 Da, und bevorzugt zwischen 20.000 - 300.000 Da, insbesondere 50.000 bis 160.000 Da, insbesondere 50.000 bis 120.000 Da und
- D: einem Zahlenmittel der Molekulargewichtsverteilung kleiner 40.000 Da, bevorzugt kleiner 35.000 Da, insbesondere kleiner 27.000 Da, besonders bevorzugt 10.000 bis 35-000 Da, insbesondere 10.000 bis 27.000 Da und

Chitosane, die sich dabei aus mindestens 60 Monomeren zusammensetzen sowie ein oder mehrere Antitranspirant-Wirkstoffe aus der Gruppe der Aluminium-Salze die Aufgaben vollständig löst

Der Anteil an Chitosan in der erfindungsgemäßen Zubereitung beträgt bevorzugt 0,001 bis 1 Gew.%, bezogen auf die Gesamtmasse der Zubereitung. Bevorzugt ist hier ein Anteil im Bereich von 0,01 bis 0,5 Gew.%, bevorzugt 0,1 bis 0,3 Gew.%, insbesondere 0,025 bis 0,3 Gew.%.

Das Chitosan oder die Chitosanderivate sind vorteilhaft maritimen Ursprungs oder enzymatisch über gentechnisch modifizierte Mikroorganismen hergestellt worden, wobei die Chitosane oder Chitosanderivate protoniert, partiell protoniert oder unprotoniert sein können. Unter Chitosanderivate werden hiermit auch die protonierten, partiell protonierten sowie unprotonierten Formen der Chitosane subsummiert.

Die erfindungsgemäß wesentlichen Parameter des Chitosans werden wie folgt bestimmt.

Die Viskosität einer 1 % Lösung wird nach Brookfield in 1 %iger Essigsäure bei 25°C gemessen.

Das Molekulargewicht wird entsprechend der GPC-Analyse in einer 0,1 molaren Nacl/0,1 Vol.%-Trifluoroessigsäure Lösung bei 23°C mit einer Säule PSS-Novema (10 µm, linear, ID 8,0 mmx300 mm), einem Fluss von 1,0 ml/min., einer Probenkonzentration von 2,0 g/l und einem Injektionsvolumen von 20 µl bestimmt.

Der Deacetylierungsgrad lässt sich über die Direkttitrationsmethode bestimmen.

Die besonders effizient wirkenden Chitosane haben ein Molekulargewicht zwischen 10.000 bis 300.000 Da, insbesondere zwischen 50.000 - 160.000 Da, ein Deacetylierungsgrad von 75 bis 98%, eine Viskosität von maximal 10 mPas, ein Zahlenmittel der Molekulargewichtsverteilung kleiner 40.000 Da und setzen sich aus mindestens 50 Monomeren zusammen.

Als besonders bevorzugt sind Chitosane mit einem Molekulargewicht kleiner 160.000 Da, einem Deacetylierungsgrad von 78 bis 86%, einer Viskosität von maximal 10 mPas und einem Zahlenmittel der Molekulargewichtsverteilung kleiner 35.000 Da.

Vorteilhaft zeigten sich insbesondere Chitosane mit einem Molekulargewicht kleiner 100.000 Da, einem Deacetylierungsgrad von 78 bis 84%, einer Viskosität von maximal 10 mPas und einem Zahlenmittel der Molekulargewichtsverteilung kleiner 27.000 Da.

Erfindungsgemäß lassen sich für den speziellen Anwendungszweck erfindunsgemäße Chitosane mit den folgenden Kombinationen der bevorzugten Parameterbereichen A, B, C und D individuell auswählen, herstellen und anwenden.

**Tabelle: Chitosanparameterbereiche**

| A | B | C | D |
|---|---|---|---|
| Deacetylierungsgrad % | Viskosität mPas | Gewichtsmittel Molekulargewichtsverteilung | Zahlenmittel Molekulargewichtsverteilung |
| | | Da | Da |
| 75 - 98 | max. 10 | < 300.000 | < 40.000 |
| 78 - 86 | 4 - 10 | < 160.000 | < 35.000 |
| 75 - 85 | 5 - 8 | < 150.000 | < 27.000 |
| 8 - 98 | | < 100.000 | 10.000 - 35.000 |
| 80 - 85 | | 20.000 - 300.000 | 10.000 - 27.000 |
| 82 - 84 | | 50.000 - 160.000 | |
| 92-98 | | 50.000 -120.000 | |

So ist beispielsweise eine Zubereitung enthaltend Chitosane mit A = 80 - 98%, B = 5 - 10 mPas, C = 50.000 - 120.000 Da und D = 10.000 - 27.000 Da ebenso bevorzugt für eine spezielle Deo/AT-Produktformulierung wie eine Zubereitung enthaltend Chitosane mit A = 82 - 84%, B = 5 - 8 mPas, C < 160.000 Da und D < 35.000 Da.

Die erfindungsgemäße spezifische Verknüpfung der bestimmten Eigenschaften der Chitosane führt dazu, dass sich sowohl stabile opake, transluzente als auch transparente Zubereitungen herstellen lassen, die mit den aus dem Stand der Technik bekannten Chitosanen nicht erhältlich sind.

Wesentlich ist dabei, dass enthaltene AT-Wirkstoffe und/oder Parfümöle durch die Zugabe von Chitosan nicht inaktierviert oder verändert werden, was es gegenüber dem Stand der Technik zu verbessern galt.

Aufgrund der guten Wasserlöslichkeit der erfindunsgemäßen Chitosane bzw. deren Derivate ist außerdem der Weißeleffekt nach Auftrag auf die Haut stark reduziert.

Überraschenderweise weisen die erfindungsgemäßen Formulierungen, umfassend die erfindungsgemäß definierten Chitosane, eine bessere, schnellere und langanhaltendere Deowirksamkeit auf als Formulierungen, die Chitosane enthalten, die diese Parameter nicht aufweisen.

Erstaunlich ist aber darüber hinaus, dass die erfindungsgemäßen Chitosane bzw. deren Derivate sich im Gegensatz zu den anderen Chitosanen, die diese Parameter nicht aufweisen, dadurch auszeichnen, dass sie in live/dead Färbungstests keine bzw. weniger als 10% getötete Bakterien aufweisen.

Dies steht den bisherigen Erkenntnissen über die Deowirksamkeit und anti-adhäsiv Wirkung von Chitosanen entgegen bzw. war bislang unklar. Bisher wurde für Chitosane im Stand der Technik nur eine antibakterielle Wirkung beschrieben. Der Vorteil der erfindungsgemäßen Chitosane und deren Derivate liegt in der Gesunderhaltung der Hautflora, da die Keime der Achselflora entgegen den Kenntnissen des Standes der Technik nicht abgetötet werden.

Zum Beleg wurden dazu In-vitro Bakterien in einem Suspensionstest mit Wirkstoffen inkubiert und Aliquots des Ansatzes mit einem live/dead Kit gefärbt und mikroskopisch ausgewertet.

Weiterhin wurden in-vivo folgende Tests durchgeführt. Nach einer Auswaschphase applizierten Probanden ein antimikrobielles Deoprodukt unter die eine Achsel und ein chitosanhaltiges, erfindungsgemäßes Deoprodukt unter die andere Achsel. Eine Stunde nach Produktauftrag wurden Achselspülungen durchgeführt und Aliquots mit dem live/dead Kit bzw. SYBR-Green II zur Auszählung der Chitosanstrukturen gefärbt und mikroskopisch ausgewertet.

Mit Hilfe des live/dead Kits können lebende und tote Bakterien unterschieden werden. Die Proben werden mit zwei verschiedenen Fluoreszenzfarbstoffen gefärbt. Syto 9 färbt dabei generell alle Zellen der Probe an (grün fluoreszierend), wohingegen Propidiumiodid nur Zellen mit beschädigter Zellmembran färbt (rot fluoreszierend). Durch ein geeignetes Mischungsverhältnis der beiden Farbstoffe kann aufgrund der verschiedenen Fluoreszenzen zwischen lebenden (grün) und toten Zellen (rot) differenziert werden.

SYBR Green II ist ein DNA spezifischer Farbstoff, der Bakterienzellen grün anfärbt und häufig zur Bestimmung des Bakterientiters herangezogen wird. Die Bakterienzellen sind nach der Färbung grün fluoreszierend. Die Chitosanstrukturen wurden mit WGA-Texas Red rot gegengefärbt, wodurch eine Bestimmung der Anzahl gebundener Bakterien an die Chitosanstrukturen erfolgen konnte.

In der live/dead Färbung zeigte sich nun erstaunlicherweise, dass alle bzw. mindestens 90% der Bakterien vital vorlagen. Somit ist das erfindungsgemäße Chitosan bzw. deren Derivate mit den spezifischen Parametern nicht für eine Zerstörung bzw. Abtötung der Bakterien verantwortlich. Vielmehr zeigte sich, dass die erfindungsgemäßen Chitosane in Kombination mit Corneozyten und Bakterien sowohl in vitro als auch in Spülungen am Probanden als Chitosanstrukturen vorliegen, an bzw. in denen 10 - 10.000, im Besonderen 10 - 1.000, Bakterien gebunden vorliegen, d.h. ein Bindung der Bakterien an bzw. in den erfindunsgemäßen Chitosanen liegt vor.

Obwohl die Bakterien in den Chitosanstrukturen zu 90% vital sind, führen diese Chitosanstrukturen dennoch dazu, dass eine bakterielle Verstoffwechselung des Schweißes nicht mehr erfolgen kann.

D.h. die erfindungsgemäßen Chitosane bzw. deren Derivate weisen keine abtötende Wirkung auf, was wiederum für die erfindungsgemäßen Zubereitungen eine Milde zur Haut verspricht, und dennoch wird eine mikrobielle Zersetzung des Schweißes zeitweilig gestoppt.

Die spezifischen Chitosane bzw. deren Derivate zeigen keine abtötende Wirkung auf Bakterien, was wiederum für die erfindungsgemäßen Zubereitungen eine Milde zur Haut verspricht, da die Hautflora in ihrem natürlichen Gleichgewicht verbleibt und so in ihrer Funktion keineswegs eingeschränkt wird.

Dadurch ergibt sich die überraschenderweise hohe Effizienz, Schnelligkeit und Dauerhaftkeit in der Deowirksamkeit der erfindungsgemäßen Formulierungen gegenüber bislang bekannten Deo/AT-Formulierungen.

Chitosane des Standes der Technik, beispielsweise mit MW über 300.000 Da, zeigen die beschriebenen Wirkung der Vitalerhaltung der Bakterien nicht.

Die Verwendung insbesondere einer O/W-Emulsion oder Gel enthaltend die erfindunsggemäßen Chitosane bzw. deren Derivate zur Schweißgeruchsreduzierung, Körpergeruchsreduzierung, zur effizienteren Deowirksamkeit und/oder Deolangzeitwirkung ist somit gewährleistet.

Langzeitwirkung bedeutet, dass chitosanhaltige Deoformulierungen nach 24 Std. eine höhere Reduktion des SNIFF-Scores erreichen als nach 4 Std. Für antimikrobielle Deoformulierungen ist im Gegensatz dazu der 4 Std. SNIFF-Score höher als der SNIFF-Score nach 24 Std.

Es ist bekannt, dass durch die Lyse der Bakterien durch antimikrobielle Wirkstoffe bakterielle Stoffwechselprodukte freigesetzt werden können, die wiederum zu Entzündungsreaktionen der Haut führen können. Durch die anti-adhäsive Wirksamkeit der erfindungsgemäßen Chitosane kann keine Freisetzung bakterieller Stoffwechselprodukte erfolgen. Zudem wird die natürliche Hautflora im Gleichgewicht gehalten, was zur Gesunderhaltung der Haut beiträgt. Zudem ist Chitosan nahezu naturidentisch mit der obersten Hautschicht, wodurch eine hohe Verträglichkeit gewährleistet wird.

Weiterer Beleg der verbesserten Deowirksamkeit erfindungsgemäßer Formulierungen zeigt sich auch im Vergleich zu herkömmlichen Deowirkstoffen, wie z.B. Ethylhexylglycerin, Methylphenylbutanol und Polyglyceryl-2-caprat. In Vergleichsversuchen wurde in-vivo die Deowirksamkeit getestet, wobei die erfindungsgemäßen Formulierungen eine zwar subjektiv bestimmte, aber dennoch bessere Wirksamkeit aufwiesen.

Sowohl in Untersuchungen der Achselflora nach Produktapplikation als auch in SNIFF-Tests konnte gezeigt werden, dass erfindungsgemäße chitosanhaltige Produkte auch eine bessere Wirksamkeit als Deoprodukte enthaltend Ethylhexylglycerin, Methylphenylbutanol und/oder Polyglyceryl-2-caprat aufweisen. Dabei ist besonders die effizientere und langanhaltende Deowirkleistung der erfindungsgemäßen Produkte hervorzuheben.

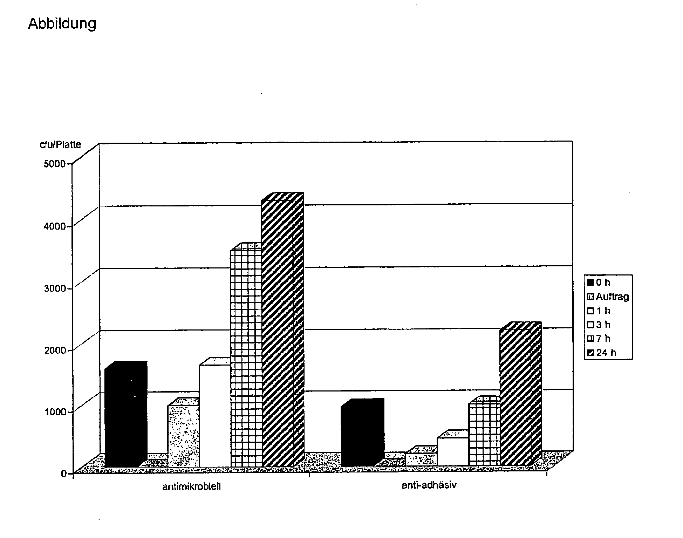

In der Abbildung 1 ist die Deowirksamkeit einer erfindungsgemäßem anti-adhäsiva Zubereitung einer antimikrobiellen Formulierung enthaltend Ethylhexylglycerin, Methylphenylbutanol und/oder Polyglyceryl-2-caprat gegenüber gestellt. Beide Produkte wurden 9 Wochen angewendet (Studie mit 15 Probanden) und zur Auswertung die Mittelwerte der keimbildenden Einheiten auf den Bakterien (cfu/Platte) ermittelt.

Die Untersuchung zeigt sehr deutlich die Reduktion der keimbildenden Einheiten der erfindunsgemäßen Zubereitung gegenüber der antimikrobiellen Formulierung und damit die effizientere und langanhaltendere Deowirkleistung der erfindungsgemäßen Produkte.

Weiterhin lassen sich überraschenderweise durch die einfache Kombination von Antitranspirantwirkstoffen, bevorzugt Aluminium-Chlorohydrate und/oder aktiviertes Aluminium-Chlorhydrat, und Chitosanen mit den aufgeführten Eigenschaften eines geringen Molekulargewichts, geringer Viskosität und hoher Deacetylierung, stabile, opake, transparente sowie transluzente kosmetische Formulierungen herstellen, die die Nachteile des Standes der Technik nicht aufweisen. Bevorzugt hat sich hierbei die Herstellung stabiler, opaker oder transparenter oder transluzenter desodorierenden Zubereitung gezeigt.

Es wird damit erstmalig vermieden, dass es durch den Einsatz von Chitosanen in Emulsionen zu Instabilitäten und/oder Eintrübungen kommt.

Insbesondere ist es möglich die emulgierenden Eigenschaften der Chitosane zu unterdrücken.

Als Antitranspirantwirkstoff lassen sich vorteilhaft saure Aluminium- und/oder Aluminium/Zirkoniumsalze in wässriger Lösung einarbeiten. Hierbei beziehen sich die beschriebenen Konzentrationsbereiche auf die so genannten Aktivgehalte der Antitranspirant-Komplexe: bei den Aluminium-Verbindungen auf wasserfreie Komplexe, bei den Aluminium/Zirkonium-Verbindungen auf wasser- und pufferfreie Komplexe. Als Puffer wird hier üblicherweise Glycin verwendet.

Die nachfolgende Auflistung vorteilhaft einzusetzender Antitranspirant-Wirker soll in keiner Weise einschränkend sein:
Aluminium-Salze (der empirischen Summenformel [Al₂(OH)ₘClₙ], wobei m+n=6):
   - Aluminiumchlorhydrat [Al₂(OH)₅Cl] x H₂O
      Standard Al-Komplexe: Locron L, Locron LIC, Locron LIF (Clariant), Chlorhydrol (Reheis), ACH-303 (Summit), Aloxicoll L (Giulini).
      Aktivierte Al-Komplexe: Reach 501 (Reheis), Aloxicoll 51 L
   - Aluminiumsesquichlorhydrat [Al₂(OH)_{4,5}Cl_{1,5}] x H₂O
      Standard Al-Komplexe: Aloxicoll 31 L (Giulini), Westchlor 186 (Westwood Chemicals)
      Aktivierte Al-Komplexe: Reach 301 (Reheis)
   - Aluminiumdichlorhydrat [Al₂(OH)₄Cl₂] x H₂O
Aluminium-Zirkonium-Salze:
   - Aluminium/Zirkonium Trichlorhydrex Glycin [Al₄Zr(OH)₁₃Cl₃] x H₂O x Gly
      Standard Al/Zr-Komplexe: Rezal 33GC (Reheis), AZG-7164 (Summit)
   - Aluminium/Zirkonium Tetrachlorhydrex Glycin [Al₄Zr(OH)₁₂Cl₄] x H₂O x Gly
      Standard Al/Zr-Komplexe: Rezal 36, Rezal 36G, Rezal 36 GC (Reheis), AZG-368 (Summit), Zirkonal L435G (Giulini), Westchlor ZR 35 BX5, Westchlor ZR 41 (Westwood Chemicals)
   - Aluminium/Zirkonium Pentachlorhydrex Glycin [Al₈Zr(OH)₂₃Cl₅] x H₂O x Gly
      Standard Al/Zr-Komplexe: Rezal 67 (Reheis), Zirkonal L540, Zirkonal L530 PG (Giulini), Westchlor ZR 80B (Westwood Chemicals)
   - Aluminium/Zirkonium Octachlorhydrex Glycin [Al₈Zr(OH)₂₀Cl₈] x H₂O x Gly:
      Westchlor ZR 82B

Ebenso vorteilhaft können aber auch Glycin-freie Aluminium/Zirkonium-Salze eingesetzt werden.

Insbesondere lassen sich durch den Zusatz an Chitosan desodorierende Zubereitungen herstellen, mit einem bevorzugtem Anteil an Antitranspirantwirkstoffen im Bereich von 1 bis 35 Gew.%, vorzugsweise von 1 bis 25 Gew. -%, besonders bevorzugt von 1 bis 20 Gew.%, bezogen auf die Gesamtmasse der Zubereitung. Bevorzugt werden Antitranspirantwirkstoffe eingesetzt, hierbei wiederum bevorzugt Aluminium Chlorohydrat (ACH), Aluminium-Zirkonium-Salze oder aktiviertes Aluminium-Chlorhydrat, ACH dabei in einer 50% wässrigen Lösung, deren Anteil dann bevorzugt im Bereich von 10 bis 50 Gew.%, insbesondere im Bereich von 10 bis 20 Gew.% bzw. 16 bis 45 Gew.%, liegt.

Überraschenderweise wirkt der Zusatz an Antitranspirantien, insbesondere ACH, dabei vorteilhaft als Lösungsvermittler für das eingesetzte Chitosan, ohne dass hierzu zusätzlich Säuren hinzugefügt werden müssen. ACH solubilisiert bzw. protoniert das unpolare Chitosan, so dass deren Wasserlöslichkeit verbessert wird.

Im Stand der Technik werden dazu extra zusätzliche Zugaben an Säure, wie Milchsäure oder Glykolsäure, vorgeschrieben, die durch die erfindungsgemäße Kombination überflüssig werden.

Die Kombination aus den spezifischen Chitosan oder Chitosanderivaten mit Aluminium-Chlorhydrat unter Vermeidung des zusätzlichen Zusatzes an Esteraseinhibitoren und/oder Säuren, ermöglicht die Herstellung stabiler, opaker oder transparenter oder transluzenter desodorierender Zubereitungen.

Insbesondere kann der Zusatz an Dicarbonsäuren und deren Ester, insbesondere Glutarsäure, Glutarsäuremonoethylester, Glutarsäurediethyl-ester, Adipinsäure, Adipinsäuremonoethylester, Adipinsäurediethylester, Malonsäure und Malonsäurediethylester, Hydroxycarbonsäuren und deren Ester, insbesondere Citronensäure, Milchsäure, Apfelsäure, Weinsäure oder Weinsäurediethylester vermieden werden.

Die erfindungsgemäßen Formulierungen umfassen daher auch Zubereitungen ohne (d.h. 0 Gew.%) eines Antitranspirant-Wirkstoffes und 0 - 20 Gew.% organische und/oder anorganische Säuren und/oder 0 - 20 Gew.% eines oder mehrerer Deodorant-Wirkstoffe, bezogen auf die Masse der Gesamtzubereitung.

Chitosan- und ACH-haltige Zubereitungen auf Emulsionsbasis des Standes der Technik wiesen häufig das Problem auf, dass an sich stabile Emulsionen durch den Zusatz an Chitosan eine gewisse Phasentrennung und/oder Eintrübung (d.h. Verlust an optischer Transparenz) aufwiesen. Dieses Problem wird in der Literatur auch als Koazervation oder Bildung wässriger Phasensysteme (APS) beschrieben (Römpp, 10. Auflage, Thieme Verlag; Koazervation, APS). Koazervation ist gemäß IUPAC-Definition die "Trennung kolloidaler Systeme in 2 flüssige Phasen".

Erstaunlicherweise weisen die erfindungsgemäßen Zubereitungen bei Zusatz von ACH diese Koazervation- oder APS-Bildung nicht auf. Ein Zusatz an Säuren ist damit überflüssig. Zudem konnte somit aufgrund des erfindungsgemäßen Einsatzes des niedermolekularem Chitosan mit hohem Deacetylierungsgrad (über 75% bzw. über 80%) eine Kompatibilitätserhöhung in Mikro-, Nano- und Makroemulsionen erzeugt werden. Es können somit höhere Konzentrationen an Chitosanen ohne Trübung oder Stabilitätsverlust in kosmetische Zubereitungen eingearbeitet werden. Bei diesen Chitosantypen handelt sich vorteilhaft um niedermolekulare Spezies, die mittels Peroxid-Behandlung hergestellt wurden. Durch die Wasserstoffperoxid-Behandlung ist eine Veränderung der Molmasse und/oder eine Quervernetzung eingetreten, die sich vorteilhaft auf die Viskosität und die Löslichkeit auswirkt.

Durch diese spezielle Art der Herstellung wird ein Chitosan erzeugt, das eine schmale Molekulargewichts- bzw. Polymerkettenlängenverteilung aufweist. Die ist besonders wichtig im Hinblick auf die Einarbeitung in transparente Mikro- und Nanoemulsionen, da bereits kleine Anteile an größeren bzw. längeren Chitosan-Ketten zu unerwünschten Eintrübungen führen würde.

Wesentlich ist, dass sich somit erstmalig transparente desodorierende Zubereitungen auf Basis von Mikro-, Nano- oder Makroemulsionen mit einem Gehalt an niedermolekularen Chitosan mit hohem Deacetylierungsrad herstellen lassen und auch transparent bleiben. Erreicht wird dies mittels gezielter Minimierung der emulgierenden Eigenschaften des Chitosans durch erfindungsgemäße Kombination der beanspruchten Molekülparameter.

Grundlage der erfindungsgemäßen Zubereitungen sind bevorzugt Mikro- und Nanoemulsionsgele beruhend auf Mikro- bzw. Nanoemulsionen vom Typ Öl-in-Wasser wie sie insbesondere in der WO 98/15255 offenbart sind.

Bevorzugt sind darüber hinaus sowohl Mikroemulsionen, Mikroemulsionsgele, Nanoemulsionen, Nanoemulsionsgele, Makroemulsionen, Makroemulsionsgele als auch alkoholische Lösungen oder Gele, die sowohl transparent, transluzent als auch opaque und niedrigviskos als auch hochviskos seien können.

Unter niedrigviskos werden dabei Viskositäten von bis zu 3000 mPas verstanden (bestimmt mittels VT-02 Viskotester der Fa. Haake mit Spindel Nr. 2 (24mm Durchmesser)).

Besonders bevorzugt sind daher Zubereitung sowohl auf Basis von transparenten alkoholischen Gele, transparenten Mikroemulsionsgelen oder transparenten Nanoemulsionsgele sowie ebenfalls auf Basis von opaquen Makroemulsionen.

Ein weiterer wesentlicher Vorteil ist, dass bei Antitranspirant(AT)-Formulierungen auf Basis von Makroemulsionen die vorzugsweise aus Fettalkoholpolyglycolether bestehen und bei AT-Mikroemulsionen die aus verzweigten Monoglyceriden und/oder verzweigten Polyoxyalkylenalkylether bestehen, durch die Zugabe des niedermolekularen Chitosans keine Wasserabscheidung oder Phasentrennung mehr beobachtet werden kann.

Erfindungsgemäße Antitranspirantformulierungen auf Basis von Makroemulsionen enthaltend Fettalkoholpolyglycolether können durch Zusatz erfindungsgemäßer Chitosane, insbesondere Chitosanen mit einem Deacetylierungsgrad von 82% bis 86%, bzw. 92-98%, einer Viskosität bis zu maximal 10 mPas, einem Gewichtsmittel der Molekulargewichtsverteilung von weniger als 160.000 Da und einem Zahlenmittel der Molekulargewichtsverteilung kleiner 40.000 Da, insbesondere kleiner 27.000 Da, die ansonsten zu beobachtende Wasserabscheidung und/oder Phasentrennung verhindern.

Ebenso wird dies durch Zusatz o.g. bevorzugter Chitosane zu Antitranspirantformulierungen auf Basis von Mikroemulsionen enthaltend verzweigte Monoglyceriden/Polyoxyalkylenalkyether erreicht. Diese Mikroemulsionen zeigen eine hohe Transparenz.

Der erfindungsgemäße Einsatz der Chitosane in Makroemulsionen enthaltend Fettalkoholpolyglycolether oder Mikroemulsionen enthaltend verzweigte Monoglyceriden/Polyoxyalkylenalkyether ist damit prädestiniert.

Vorteilhaft können erfindungsgemäßen Zubereitungen auch Desodorantien zugesetzt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluss selbst wird dadurch nicht beeinflusst, im Idealfalle wird nur die mikrobielle Zersetzung des Schweißes zeitweilig gestoppt, wie es erfindungsgemäß gelingt. Auch die Kombination von Adstringentien mit antimikrobiell wirksamen Stoffen in ein und derselben Zusammensetzung ist gebräuchlich.

Alle für Desodorantien gängigen Wirkstoffe können vorteilhaft genutzt werden, beispielsweise Geruchsüberdecker wie die gängigen Parfüm bestandteile, Geruchsabsorber, beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet in die erfindungsgemäßen Zubereitungen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden.

Die Menge der Desodorantien (eine oder mehrere Verbindungen) in den Zubereitungen beträgt vorzugsweise 0,01 bis 10 Gew.%, bevorzugt 0,05 bis 5 Gew.% bezogen auf das Gesamtgewicht der Zubereitung.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, UV-Filter, Antioxidantien, wasserlösliche Vitamine, Mineralstoffe, suspendierte Festkörperpartikel, Parfüme, Substanzen zum Verhindern des Schäumens, Farbstoffe, Pigmente, die eine färbende Wirkung haben, Verdickungsmittel, anfeuchtende und/oder feuchthaltende Substanzen oder andere übliche Bestandteile einer kosmetischen Formulierung wie Alkohole, Polyole, Polymere, Schaumstabilisatoren oder Silikonderivate.

Zusammenfassend weisen die erfindungsgemäßen Zubereitungen folgende Vorteile auf:
- Erhalt mindestens 90% lebende Bakterien, ermittelt durch live/dead Tests - D.h. die Gesunderhaltung der natürlichen Hautflora ist im Gegensatz zu herkömmlichen Deoprodukten gewährleistet. Des Weiteren werden keine bakteriellen Stoffwechselprodukte durch die Lyse der Bakterienzellen freigesetzt, die zu Entzündungsreaktionen führen können.
- kein Abtöten der Bakterien. Die Bakterien werden nicht abgetötet, sondern von den Corneozyten abgelöst und an und/oder in Chitosanstrukturen gebunden
- hohe Effizienz in der Deowirksamkeit
- stabile, transparente kosmetische Zubereitungen

Die erindungsgemäßen Chitosane und/oder deren Derivate lassen sich somit erstmals zur An- oder Einbindung von bis zu 90% vitaler, gram positiver Bakterien, in kosmetischen Zubereitungen verwenden.

Mit den erfindungsgemäßen Zubereitungen lassen sich häufig auftretende unerwünschte physiko-chemische Phänomene in O/W-Emulsionen und alkoholischen Gelen, wie die Koazervation und/oder Bildung wässriger Phasensysteme (APS) vermeiden.

Die erfindunsgemäßen Zubereitungen dienen darüber hinaus bevorzugt zur Stabilisierung, d.h. Verhinderung der Phasentrennung in opaquen Systemen und Eintrübung bzw. Verlust an optischer Transparenz in transparenten und transluzenten Systemen.

Zur Applikation der Zubereitung lassen sich herkömmliche Packmittel für Deodorantien und/oder Antitransipirantien verwenden, z. B. Stiftdispenser, Geldispenser, Tuben, Roller und Zerstäuber.

Besonders bevorzugt sind die transparenten Lösungen oder Gele in Deo-Roll-on zu verwenden.

In den nachfolgenden Beispielen beziehen sich die Angaben in Gewichtsprozent auf die Gesamtmasse der Zubereitung. Die hierin genannten Chitosane oder Chitosanderivate weisen entsprechende Eigenschaften und Merkmale auf, wie sie in den Ansprüche beschrieben sind.

### Beispiele

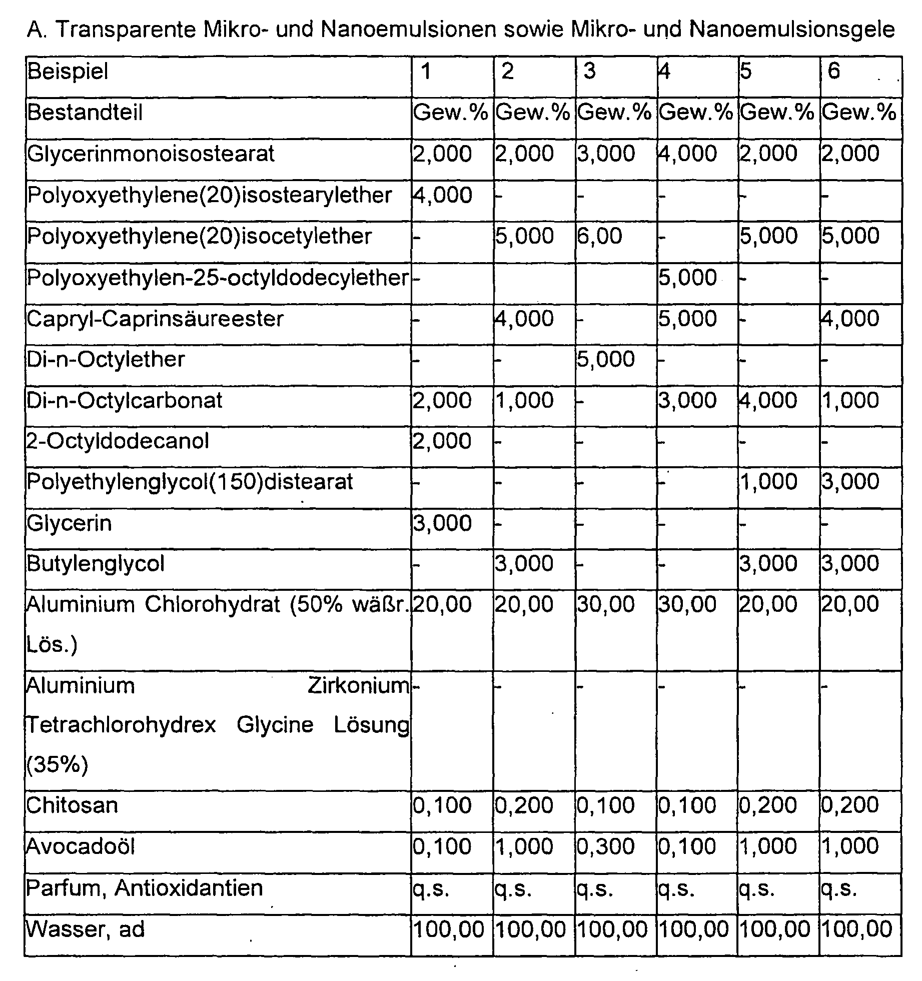

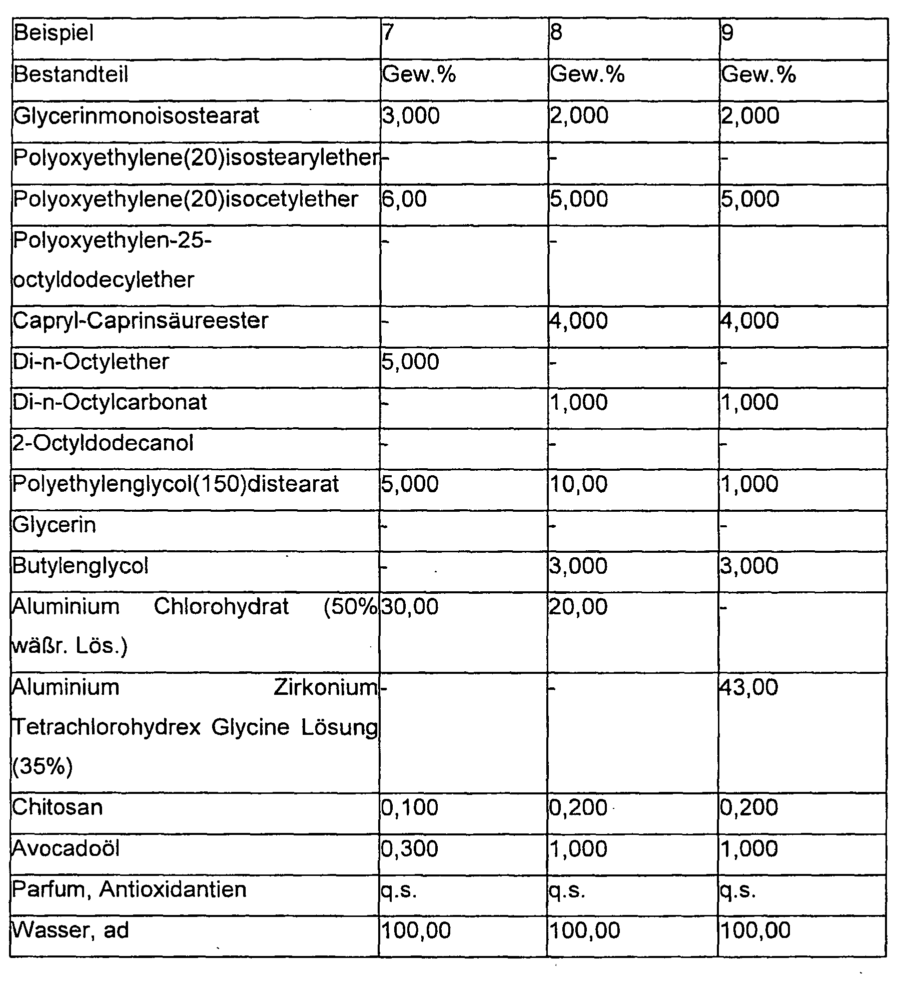

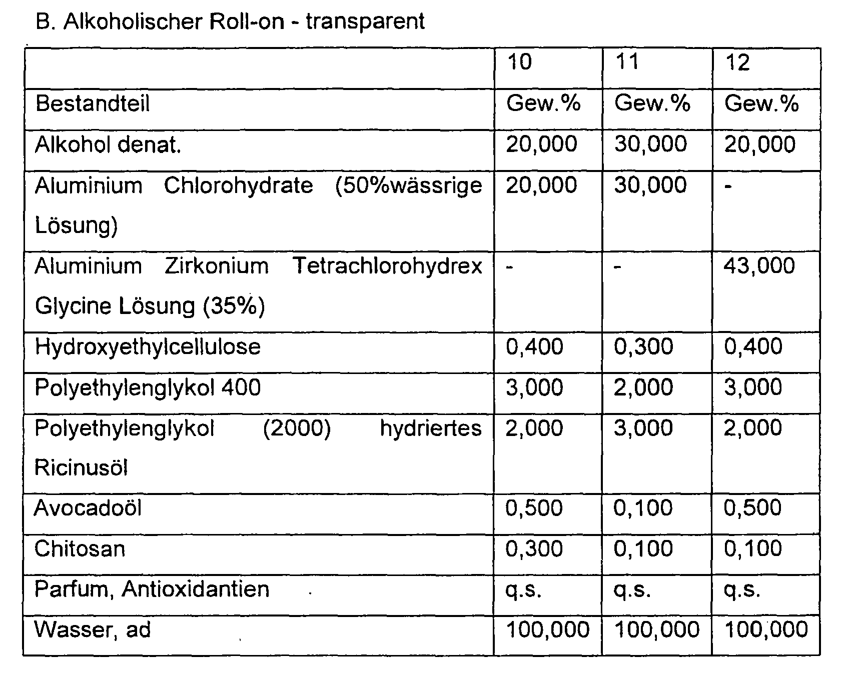

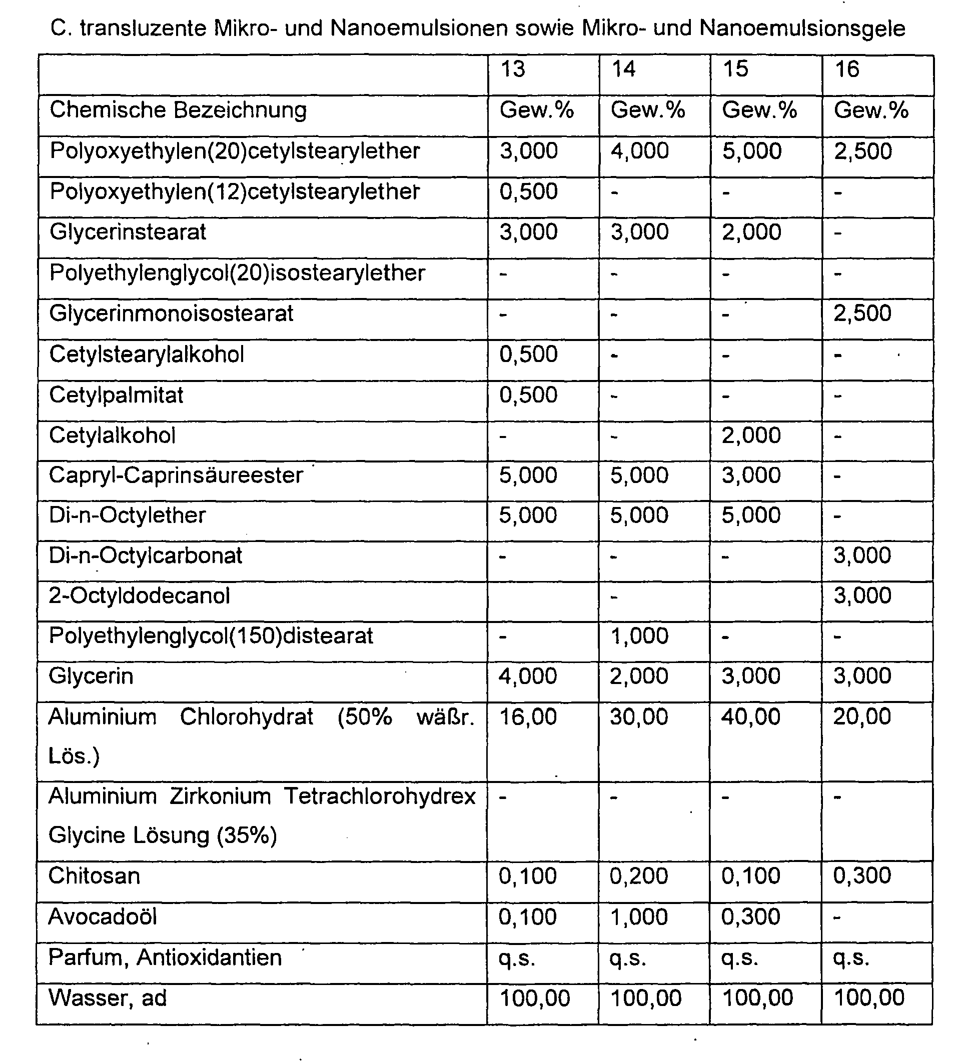

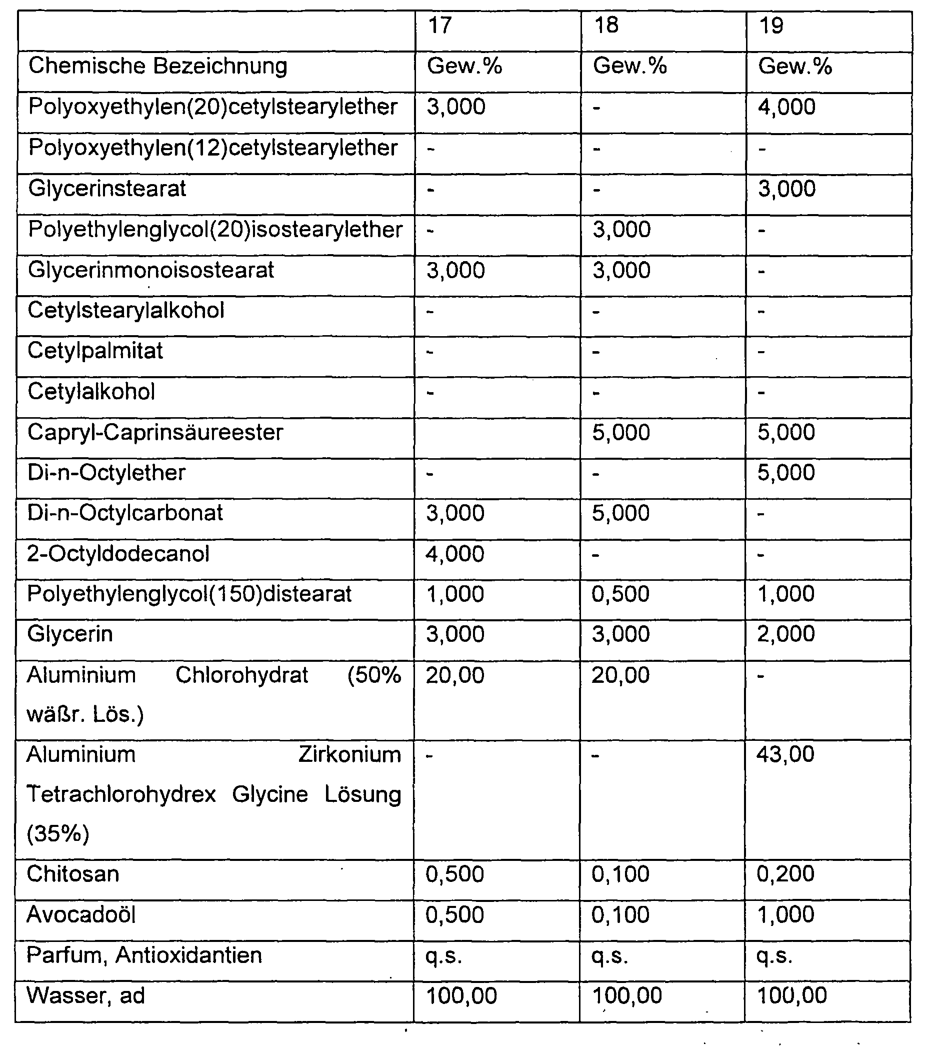

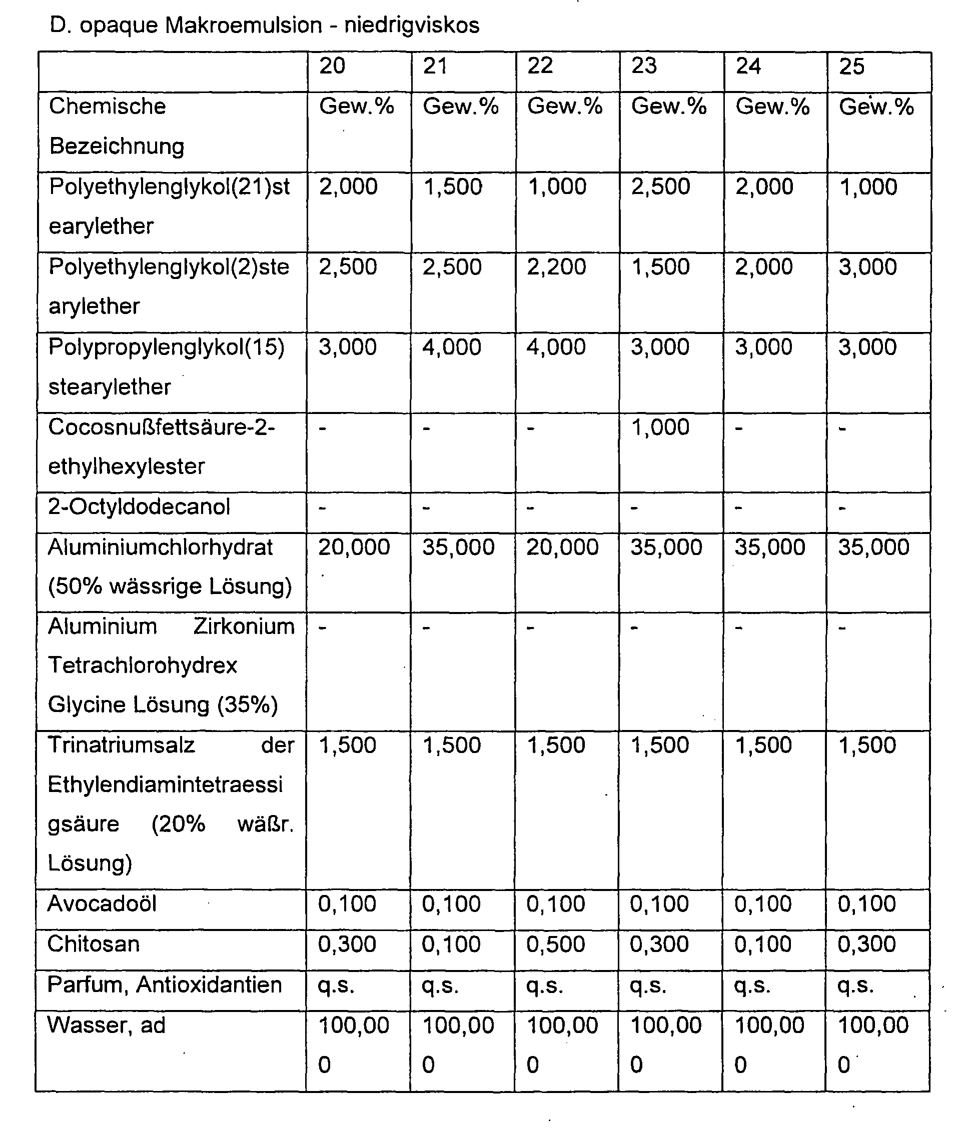

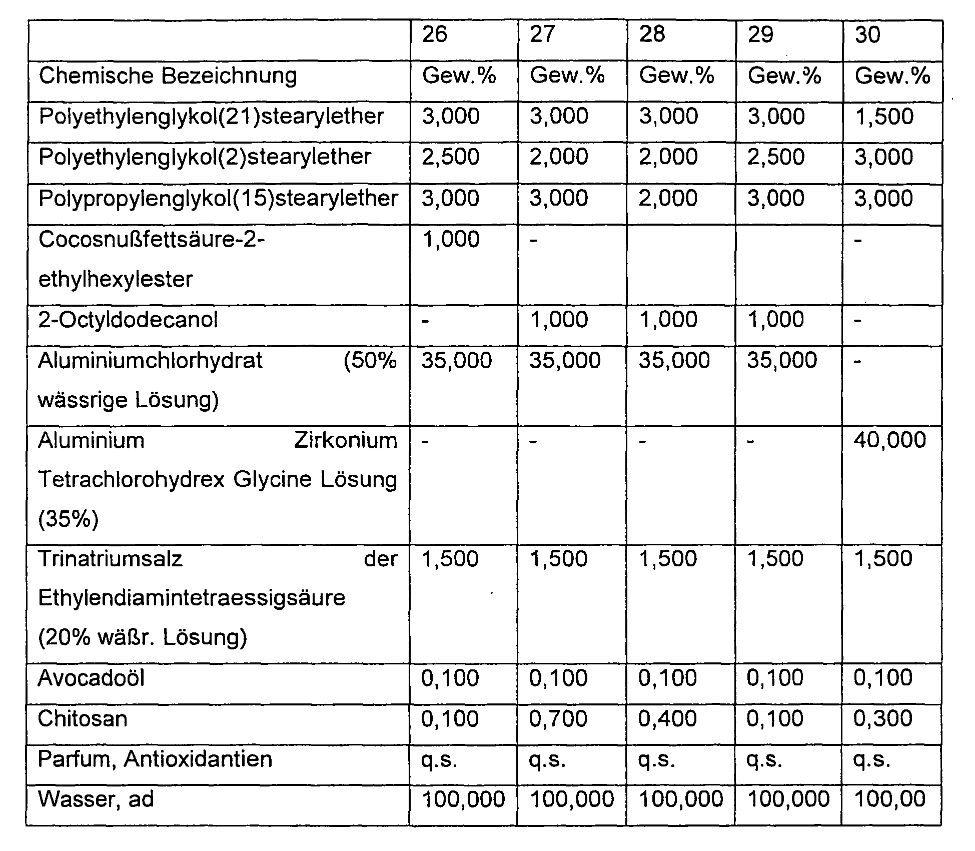

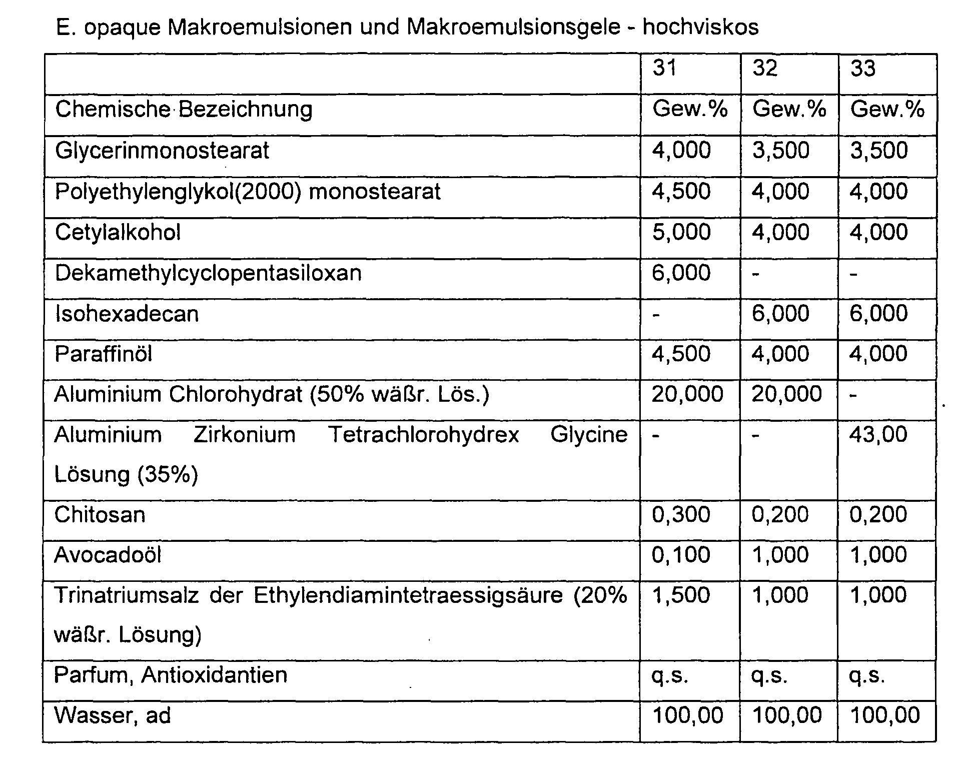

## Patentansprüche

1. Kosmetische Zubereitung umfassend Chitosan oder Chitosanderivate mit
- einem Deacetylierungsgrad von 75% bis 98%,
- einer Viskosität bis zu maximal 10 mPas,
- einem Gewichtsmittel der Molekulargewichtsverteilung von 10.000 bis 300.000 Da,
- einem Zahlenmittel der Molekulargewichtsverteilung kleiner 40.000 Da und
- sie setzen sich aus mindestens 50 Monomeren zusammen sowie
ein oder mehrere Antitranspirant-Wirkstoffe aus der Gruppe der Aluminium-Salze,

2. Kosmetische Zubereitung nach Anspruch 1 umfassend Chitosan oder Chitosanderivate mit
- einem Deacetylierungsgrad von 80 bis 98%,
- einem Gewichtsmittel der Molekulargewichtsverteilung von weniger als 160.000 Da, und
- einem Zahlenmittel der Molekulargewichtsverteilung kleiner 35.000.

3. Zubereitung nach Anspruch 1, **dadurch gekennzeichnet, dass** Kombinationen der bevorzugten Parameterbereichen Deacetylierungsgrad (A), Viskosität (B), Gewichtsmittel Molekulargewichtsverteilung (C) und Zahlenmittel Molekular-gewichtsverteilung (D) gewählt werden aus
A [%]: 75 - 98, 78 - 86, 75 - 85, 80 - 98, 80 - 85, 82 - 84 oder 92-98 und
B [mPas]: <10, 4 -10 oder 5 - 8 und
C [Da]: < 300.000, < 160.000, < 150.000, < 100.000, 20.000 - 300.000, 50.000 - 160.000 oder 50.000 -120.000 und
D [Da]: < 40.000, < 35.000, < 27.000, 10.000 - 35.000 oder 10.000 - 27.000.

4. Zubereitung nach einem der vorstehenden Anspruche umfassend Chitosan oder Chitosanderivate mit einem Deacetylierungsgrad von 92 bis 98%.

5. Zubereitung nach einem der vorstehenden Ansprüche umfassend Chitosan oder Chitosanderivate in einer Menge von 0,001 bis 1 Gew.%, bevorzugt 0,01 bis 0,5 Gew.%, besonders bevorzugt 0,1 bis 0,3 Gew.%, insbesondere 0,025 bis 0,3 Gew.%, bezogen auf die Gesamtmasse der Zubereitung.

6. Zubereitung nach Anspruch 1, **dadurch gekennzeichnet, dass** der oder die Antitranspirant-Wirkstoffe in einer Menge von 1 bis 35 Gew.%, vorzugsweise von 1 bis 25 Gew. -%, besonders bevorzugt von 1 bis 20 Gew.%, bezogen auf die Gesamtmasse der Formulierung, zugesetzt sind.

7. Zubereitung nach Anspruch 1 enthaltend 0 Gew.% Esteraseinhibitoren und/oder Sauren, bezogen auf die Gesamtmasse der Zubereitung.

8. Zubereitung nach Anspruch 7, **dadurch gekennzeichnet, dass** 0 Gew.% Dicarbonsäuren und deren Ester, insbesondere Glutarsäure, Glutarsäuremonoethylester, Glutarsäurediethyl-ester, Adipinsäure, Adipinsäuremonoethylester, Adipinsaurediethyloster, Malonsäure und Malonsäurediethylester, Hydroxycarbonsäuren und deren Ester, insbesondere Citronensaure, Milchsäure, Apfelsäure, Weinsaure oder Weinsäurediethylester enthalten sind.

9. Zubereitung nach einem der vorstehenden Ansprüche, **dadurch gekennzeichnet, dass** Avocadoöl, bevorzugt zu einem Anteil von bis zu 0,5 Gew.%, insbesondere 0,1 Gew.%, bezogen auf die Gesamtmasse der Zubereitung, enthalten ist.

10. Zubereitung nach einem der vorstehenden Ansprüche, **dadurch gekennzeichnet, dass** die Zubereitung transparent, transluzent oder opaque ist.

11. Zubereitung nach Anspruch 16 auf Basis von transparenten alkoholischen Gelen oder Lösungen, transparenten Mikroemulsionen oder Mikroemulsionsgelen oder transparenten Nanoemulsionen oder Nanoemulsionsgelen.

12. Zubereitung nach Anspruch 11 enthaltend verzweigte Monoglyceride und/oder verzweigte Polyoxyalkylenalkylether.

13. Zubereitung nach Anspruch 10 auf Basis von transluzenten oder opaquen Makroemulsionen.

14. Zubereitung nach Anspruch 13 auf Basis von Makroemulsionen enthaltend Fettalkoholpolyglycolether.

15. Verwendung einer Zubereitung nach einem der Ansprüche 1 bis 14 in einem Deo Roll-on, Zerstäuber, Stiftdispenser, Geldispenser oder Tube.

16. Verwendung der Kombination aus Chitosan und Aluminium-Salze, entsprechend Anspruch 1 bis 14 zur Herstellung einer stabilen, opaken oder transparenten oder transluzenten desodorierende Zubereitung.

17. Verwendung der Kombination nach Anspruch 16 in emulsionsbasierenden oder alkoholischen Zubereitungen zur Vermeidung der Koazervation und/oder Bildung wässriger Phasensysteme (APS).

18. Verwendung der Kombination nach Anspruch 16 zur Vermeidung von Instabilitäten und/oder Eintrobungen von emulsionsbasierenden kosmetischen Zubereitungen.

19. Verwendung einer Zubereitung nach einem der Ansprüche 1 bis 14 zur Stabilisierung und Verhinderung von Phasentrennung in opaquen Systemen oder Verhinderung der Eintrübung oder Verlust an optischer Transparenz in transparenten oder transluzenten Systemen.

## Claims

1. Cosmetic preparation comprising chitosan or chitosan derivatives with
- a degree of deacetylation of from 75% to 98%,
- a viscosity up to a maximum of 10 mPas,
- a weight-average molecular weight distribution of from 10 000 to 300 000 Da,
- a number-average molecular weight distribution of less than 40 000 Da and
- they are composed of at least 50 monomers, and
one or more antiperspirant active ingredients from the group of aluminium salts.

2. Cosmetic preparation according to Claim 1, comprising chitosan or chitosan derivatives with
- a degree of deacetylation of from 80 to 98%,
- a weight-average molecular weight distribution of less than 160 000 Da, and
- a number-average molecular weight distribution of less than 35 000.

3. Preparation according to Claim 1, **characterized in that** combinations of the preferred parameter ranges degree of deacetylation (A), viscosity (B), weight-average molecular weight distribution (C) and number-average molecular weight distribution (D) are selected from
A [%]: 75 - 98, 78 - 86, 75 85, 80 - 98, 80 - 85, 82 - 84 or 92 - 98 and
B [mPas] : < 10, 9 - 10 or 5 - 8 and
C [Da]: < 300 000, < 160 000, < 150 000, < 100 000, 20 000 - 300 000, 50 000 - 160 000 or 50 000 - 120 000 and
D [Da]: < 40 000, < 35 000, < 27 000, 10 000 - 35 000 or 10 000 - 27 000.

4. Preparation according to one of the preceding claims, comprising chitosan or chitosan derivatives with a degree of deacetylation of from 92 to 98%.

5. Preparation according to one of the preceding claims, comprising chitosan or chitosan derivatives in an amount of from 0.001 to 1% by weight, preferably 0.01 to 0.5% by weight, particularly preferably 0.1 to 0.3% by weight, in particular 0.025 to 0.3% by weight, based on the total mass of the preparation.

6. Preparation according to Claim 1, **characterized in that** the antiperspirant active ingredient or ingredients are added in an amount of from 1 to 35% by weight, preferably from 1 to 25% by weight, particularly preferably from 1 to 20% by weight, based on the total mass of the formulation.

7. Preparation according to Claim 1, comprising 0% by weight of esterase inhibitors and/or acids, based on the total mass of the preparation.

8. Preparation according to Claim 7, **characterized in that** 0% by weight of dicarboxylic acids and esters thereof, in particular glutaric acid, monoethyl glutarate, diethyl glutarate, adipic acid, monoethyl adipate, diethyl adipate, malonic acid and diethyl malonate, hydroxycarboxylic acids and esters thereof, in particular citric acid, lactic acid, maleic acid, tartaric acid or diethyl tartrate are present.

9. Preparation according to one of the preceding claims, **characterized in that** avocado oil is present, preferably in a fraction of up to 0.5% by weight, in particular 0.1% by weight, based on the total mass of the preparation.

10. Preparation according to one of the preceding claims, **characterized in that** the preparation is transparent, translucent or opaque.

11. Preparation according to Claim 16 based on transparent alcoholic gels or solutions, transparent microemulsions or microemulsion gels or transparent nanoemulsions or nanoemulsion gels.

12. Preparation according to Claim 11, comprising branched monoglycerides and/or branched polyoxyalkylene alkyl ethers.

13. Preparation according to Claim 10 based on translucent or opaque macroemulsions.

14. Preparation according to Claim 13 based on macroemulsions comprising fatty alcohol polyglycol ethers.

15. Use of a preparation according to one of Claims 1 to 14 in a deodorant roll-on, atomizer, stick dispenser, gel dispenser or tube.

16. Use of the combination of chitosan and aluminium salts, corresponding to Claims 1 to 14, for producing a stable, opaque or transparent or translucent deodorizing preparation.

17. Use of the combination according to Claim 16 in emulsion-based or alcoholic preparations for avoiding the coacervation and/or formation of aqueous phase systems (APS).

18. Use of the combination according to Claim 16 for avoiding instabilities and/or clouding of emulsion-based cosmetic preparations.

19. Use of a preparation according to one of Claims 1 to 14 for the stabilization and prevention of phase separation in opaque systems or preventing clouding or loss of optical transparency in transparent or translucent systems.

## Revendications

1. Composition cosmétique comprenant du chitosane ou des dérivés de chitosane présentant
- un degré de désacétylation de 75% à 98%,
- une viscosité d'au maximum 10 mPa.s,
- une moyenne pondérale de la répartition des poids moléculaires de 10 000 à 300 000 Da,
- une moyenne numérique de la répartition des poids moléculaires inférieure à 40 000 Da et
- constitués par au moins 50 monomères ainsi que
une ou plusieurs substances actives antiperspirantes du groupe des sels d'aluminium.

2. Composition cosmétique selon la revendication 1 comprenant du chitosane ou des dérivés de chitosane présentant
- un degré de désacétylation de 80 à 98%,
- une moyenne pondérale de la répartition des poids moléculaires inférieure à 160 000 Da et
- une moyenne numérique de la répartition des poids moléculaires inférieure à 35 000 Da.

3. Composition selon la revendication 1, **caractérisée en ce que** des combinaisons des plages préférées des paramètres degré de désacétylation (A), viscosité (B), moyenne pondérale de la répartition des poids moléculaires (C) et moyenne numérique de la répartition des poids moléculaires (D) sont choisies parmi
A [%] : 75-98, 78-86, 75-85, 80-98, 80-85, 82-84 ou 92-98 et
B[mPa.s] : < 10,4-10 ou 5-8 et
C [Da] : < 300 000, < 160 000, < 150 000, < 100 000, 20 000-300 000, 50 000-160 000 ou 50 000-120 000 et
D [Da] : < 40 000, < 35 000, < 27 000, 10 000-35 000 ou 10 000-27 000.

4. Composition selon l'une quelconque des revendications précédentes, comprenant du chitosane ou des dérivés de chitosane présentant un degré de désacétylation de 92 à 98%.

5. Composition selon l'une quelconque des revendications précédentes, comprenant du chitosane ou des dérivés de Chitosane en une quantité de 0,001 à 1% en poids, de préférence de 0,01 à 0,5% en poids, de manière particulièrement préférée de 0,1 à 0,3% en poids, en particulier de 0,025 à 0,3% en poids, par rapport à la masse totale de la composition.

6. Composition selon la revendication 1, **caractérisée en ce que** la ou les substances actives antiperspirantes sont ajoutées en une quantité de 1 à 35% en poids, de préférence de 1 à 25% en poids, de manière particulièrement préférée de 1 à 20% en poids, par rapport à la masse totale de la formulation.

7. Composition selon la revendication 1 contenant 0% en poids d'inhibiteurs d'estérase et/ou d'acides, par rapport à la masse totale de la composition.

8. Composition selon la revendication 7, **caractérisée en ce qu'**elle contient 0% en poids d'acides dicarboxyliques et de leurs esters, en particulier l'acide glutarique, l'ester monoéthylique de l'acide glutamique, l'ester diéthylique de l'acide glutarique, l'acide adipique, l'ester monoéthylique de l'acide adipique, l'ester diéthylique de l'acide adipique, l'acide malonique et l'ester diéthylique de l'acide malonique, d'acides hydroxycarboxyliques et de leurs esters, en particulier l'acide citrique, l'acide lactique, l'acide malique, l'acide tartrique ou l'ester diéthylique de l'acide tartrique.

9. Composition selon l'une quelconque des revendications précédentes, **caractérisée en ce qu'**elle contient de l'huile d'avocat, de préférence à raison d'une proportion allant jusqu'à 0,5% en poids, en particulier 0,1% en poids, par rapport à la masse totale de la composition.

10. Composition selon l'une quelconque des revendications précédentes, **caractérisée en ce que** à composition est transparente, translucide ou opaque.

11. Composition selon la revendication 16 à base de gels ou des solutions alcooliques transparent(e)s, de microémulsions ou de gels de microémulsions transparent(e)s ou de nanoémulsions ou de gels de nanoémulsions transparent(e)s.

12. Composition selon la revendication 11, contenant des monoglycérides ramifiés et/ou des polyoxyalkylénalkyléthers ramifiés.

13. Composition selon la revendication 10 à base de macroémulsions translucides ou opaques.

14. Composition selon la revendication 13 à base de macroémulsions contenant des polyglycoléthers d'alcools gras.

15. Utilisation d'une composition selon l'une quelconque des revendications 1 à 14 dans un déodorant dans un réservoir à bille, un pulvérisateur, un distributeur en bâton, un distributeur de gel ou un tube.

16. Utilisation de la combinaison de chitosane et de sels d'aluminium, correspondant à la revendication 1 à 14 pour la préparation d'une composition désodorisante stable, opaque ou transparente ou translucide.

17. Utilisation de la combinaison selon la revendication 16 dans des compositions à base d'émulsion ou alcooliques pour éviter la coacervation et/ou la formation de systèmes de phases aqueuses (APS).

18. Utilisation de la combinaison selon la revendication 16 pour éviter les instabilités et/ou les troubles de compositions cosmétiques à base d'émulsions.

19. Utilisation d'une composition selon l'une quelconque des revendications 1 à 14 pour stabiliser et éviter la séparation de phases dans des systèmes opaques ou éviter un trouble ou une perte de transparence optique dans des systèmes transparents ou translucides.
